# EUROPEAN PATENT APPLICATION

(11) **EP 4 742 265 A1**
(43) Date of publication of application: **13.05.2026**
(21) Application number: 25213382.2
(22) Date of filing: 04.11.2025
(51) Int. Cl.: G16H 40/20, G16H 40/40, G16H 40/63

(54) **MONITOR SETTING METHOD AND APPARATUS, ELECTRONIC DEVICE, STORAGE MEDIUM, AND PROGRAM PRODUCT**

(30) Priority: 08.11.2024 CN 202411590097
(71) Applicant: GE Precision Healthcare LLC, Waukesha, WI 53188 (US)
(72) Inventor: DELZAC, Pierre, 78533 Buc (FR); HUI, Hui, Wuxi (CN); WANG, Qi, Wuxi (CN); ZHOU, Puzhen, Wuxi (CN)
(74) Representative: Kilburn & Strode LLP

(57) **Abstract**

The present disclosure relates to a monitor setting method and apparatus, an electronic device, a storage medium, and a program product. The method includes: checking a connection state between a monitor and a dock device; in response to detecting that the monitor is connected to the dock device, automatically reading device information of the dock device from a memory of the dock device; determining, on the basis of the device information of the dock device, that the dock device has been replaced, and automatically generating a location information determination interface; acquiring current location information of the monitor from the location information determination interface; and acquiring target setting information of the monitor matching the current location information, and setting the monitor according to the target setting information.

## Description

### TECHNICAL FIELD

The present disclosure relates to the technical field of medical devices, and in particular, to a monitor setting method, a monitor setting apparatus, an electronic device, a computer-readable storage medium, and a computer program product.

### BACKGROUND

In the current medical care environment, patient transport is one of the common and vital clinical work procedures within hospitals. In this procedure, a patient is typically moved from one care unit to another care unit by using a portable monitor, for further treatment or monitoring. In the related art, when a patient is transported from one care unit (e.g., a general ward) to another care unit (e.g., an intensive care unit), medical personnel need to manually set a monitor to satisfy requirements of the new care unit.

### SUMMARY

The present disclosure provides a technical solution for setting a monitor.

According to an aspect of the present disclosure, provided is a monitor setting method, comprising:
checking a connection state between a monitor and a dock device;
in response to detecting that the monitor is connected to the dock device, automatically reading device information of the dock device from a memory of the dock device;
determining, on the basis of the device information of the dock device, that the dock device has been replaced, and automatically generating a location information determination interface;
acquiring current location information of the monitor from the location information determination interface; and
acquiring target setting information of the monitor matching the current location information, and setting the monitor according to the target setting information.

In a possible implementation, the current location information comprises:
identification information of a current care unit in which the monitor is located;
   or,
the identification information of the current care unit in which the monitor is located, and identification information of a current bed of a target patient corresponding to the monitor.
In a possible implementation, the target setting information comprises:
a target monitoring mode of the monitor.

In a possible implementation, the acquiring current location information of the monitor comprises:
acquiring location information of the dock device; and
determining the current location information of the monitor according to the location information of the dock device.

In a possible implementation, the acquiring location information of the dock device comprises:
reading the location information of the dock device from the memory;
   or,
determining the location information of the dock device according to the device information of the dock device and a device information and location mapping table, wherein the device information and location mapping table is acquired from a server end.

In a possible implementation, the acquiring current location information of the monitor from the location information determination interface comprises:
acquiring the current location information of the monitor inputted or selected by a user in the location information determination interface.

In a possible implementation, before the acquiring the current location information of the monitor inputted or selected by a user in the location information determination interface, the method further comprises:
displaying, in the location information determination interface, identification information of the last care unit in which the monitor was located;
   or,
displaying, in the location information determination interface, the identification information of the last care unit in which the monitor was located and identification information of a bed to be confirmed, wherein the bed to be confirmed is automatically allocated by a server end from among free beds.

In a possible implementation, after the acquiring the current location information of the monitor inputted or selected by a user in the location information determination interface, the method further comprises:
determining the location information of the dock device according to the current location information;
storing, in the memory, the location information of the dock device; and/or recording, in the device information and location mapping table, a mapping relationship between the device information and the location information of the dock device, and uploading the updated device information and location mapping table to the server end.

In a possible implementation, the acquiring target setting information of the monitor matching the current location information comprises:
reading setting information corresponding to the dock device from the memory, and determining the target setting information of the monitor according to the setting information corresponding to the dock device;
   or,
determining, according to the current location information and a location and setting information mapping table, the target setting information of the monitor matching the current location information, wherein the location and setting information mapping table is acquired from the server end.

In a possible implementation, the acquiring target setting information of the monitor matching the current location information comprises:
displaying a setting information input interface; and
acquiring the target setting information of the monitor inputted or selected by the user in the setting information input interface.

In a possible implementation, the displaying a setting information input interface comprises:
in response to the setting information corresponding to the dock device not being present in the memory and/or setting information matching the current location information not being present in the location and setting information mapping table, displaying the setting information input interface.

In a possible implementation, after the acquiring the target setting information of the monitor inputted or selected by the user in the setting information input interface, the method further comprises:
determining, according to the target setting information, the setting information corresponding to the dock device, and storing, in the memory, the setting information corresponding to the dock device;
   and/or,
recording, in the location and setting information mapping table, a mapping relationship between the current location information and the target setting information, and uploading the updated location and setting information mapping table to the server end.

In a possible implementation, the method further comprises:
in response to a state of the target patient corresponding to the monitor being changed to discharged, setting the monitor according to default setting information.

In a possible implementation, the method further comprises:
determining, on the basis of the device information of the dock device, that the dock device has not been replaced, and skipping generating the location information determination interface.

According to an aspect of the present disclosure, provided is a monitor setting apparatus, comprising:
a check module, configured to check a connection state between a monitor and a dock device;
a reading module, configured to, in response to detecting that the monitor is connected to the dock device, automatically read device information of the dock device from a memory of the dock device;
a generation module, configured to determine, on the basis of the device information of the dock device, that the dock device has been replaced, and automatically generate a location information determination interface;
an acquisition module, configured to acquire current location information of the monitor from the location information determination interface; and
a setting module, configured to acquire target setting information of the monitor matching the current location information, and set the monitor according to the target setting information.

In a possible implementation, the current location information comprises:
identification information of a current care unit in which the monitor is located;
   or,
the identification information of the current care unit in which the monitor is located, and identification information of a current bed of a target patient corresponding to the monitor.

In a possible implementation, the target setting information comprises:
a target monitoring mode of the monitor.

In a possible implementation, the acquisition module is configured to:
acquire location information of the dock device; and
determine the current location information of the monitor according to the location information of the dock device.

In a possible implementation, the acquisition module is configured to:
read the location information of the dock device from the memory;
   or,
determine the location information of the dock device according to the device information of the dock device and a device information and location mapping table, wherein the device information and location mapping table is acquired from a server end.

In a possible implementation, the acquisition module is configured to:
acquire the current location information of the monitor inputted or selected by a user in the location information determination interface.

In a possible implementation, the apparatus further comprises a display module, and the display module is configured to:
display, in the location information determination interface, identification information of the last care unit in which the monitor was located;
   or,
display, in the location information determination interface, the identification information of the last care unit in which the monitor was located and identification information of a bed to be confirmed, wherein the bed to be confirmed is automatically allocated by a server end from among free beds.

In a possible implementation, the apparatus further comprises:
a determination module, configured to determine the location information of the dock device according to the current location information; and
a first storage module, configured to store, in the memory, the location information of the dock device; and/or record, in the device information and location mapping table, a mapping relationship between the device information and the location information of the dock device, and upload the updated device information and location mapping table to the server end.

In a possible implementation, the setting module is configured to:
read setting information corresponding to the dock device from the memory, and determine the target setting information of the monitor according to the setting information corresponding to the dock device;
   or,
determine, according to the current location information and a location and setting information mapping table, the target setting information of the monitor matching the current location information, wherein the location and setting information mapping table is acquired from the server end.

In a possible implementation, the setting module is configured to:
display a setting information input interface; and
acquire the target setting information of the monitor inputted or selected by the user in the setting information input interface.

In a possible implementation, the setting module is configured to:
in response to the setting information corresponding to the dock device not being present in the memory and/or setting information matching the current location information not being present in the location and setting information mapping table, display the setting information input interface.

In a possible implementation, the apparatus further comprises a second storage module, and the second storage module is configured to:
determine, according to the target setting information, the setting information corresponding to the dock device, and store, in the memory, the setting information corresponding to the dock device;
   and/or,
record, in the location and setting information mapping table, a mapping relationship between the current location information and the target setting information, and upload the updated location and setting information mapping table to the server end.

In a possible implementation, the setting module is further configured to:
in response to a state of the target patient corresponding to the monitor being changed to discharged, set the monitor according to default setting information.

In a possible implementation, the generation module is further configured to:
determine, on the basis of the device information of the dock device, that the dock device has not been replaced, and skip generating the location information determination interface.

According to an aspect of the present disclosure, provided is an electronic device, comprising: one or more processors; and a memory for storing executable instructions, wherein the one or more processors are configured to invoke the executable instructions stored by the memory to perform the above method.

According to an aspect of the present disclosure, provided is a computer-readable storage medium, having computer program instructions stored thereon, wherein the computer program instructions, when executed by a processor, implement the above method.

According to an aspect of the present disclosure, provided is a computer program product, comprising computer-readable code or a non-volatile computer-readable storage medium bearing computer-readable code, wherein when the computer-readable code is run in an electronic device, a processor in the electronic device performs the above method.

In the embodiments of the present disclosure, a connection state between a monitor and a dock device is checked. In response to detecting that the monitor is connected to the dock device, device information of the dock device is automatically read from a memory of the dock device. It is determined, on the basis of the device information of the dock device, that the dock device has been replaced, and a location information determination interface is automatically generated. Current location information of the monitor is acquired from the location information determination interface. Target setting information of the monitor matching the current location information is acquired, and the monitor is set according to the target setting information. In this way, the connection state between the monitor and the dock device is automatically checked, and a change in location is intelligently determined on the basis of the device information of the dock device. The location information determination interface is automatically generated for confirmation performed by a user, so that settings of the monitor are automatically adjusted. The embodiments of the present disclosure significantly reduce manual intervention by the user, reduce the risk of operational error, improve the efficiency and accuracy of changing of monitor settings during patient transport, and optimize clinical work procedures.

In the embodiments of the present disclosure, the dock device does not need a system unit (e.g., a central processing unit, or CPU). Thus, manufacturing costs of the dock device can be significantly reduced, which means that medical institutions can purchase more dock devices to satisfy clinical requirements without bearing an overly heavy economic burden. In addition, the design free of system units further reduces the complexity and maintenance difficulties of the dock device, thereby reducing long-term operation costs.

In addition, the dock device free of any system unit can be easily integrated with different medical devices or systems, thereby improving interconnectivity and interoperability of medical devices. A medical institution can flexibly configure and upgrade a medical device according to actual requirements to satisfy requirements of different departments or patients.

It should be understood that the foregoing general description and the following detailed description are merely exemplary and explanatory and do not limit the present disclosure.

Other features and aspects of the present disclosure will become apparent from the following detailed description of exemplary embodiments provided with reference to the accompanying drawings.

### BRIEF DESCRIPTION OF THE DRAWINGS

The accompanying drawings here are incorporated into the specification, constitute a part of the specification, illustrate embodiments compliant with the present disclosure, and are used together with the specification to describe the technical solutions of the present disclosure.
FIG. 1 shows a flowchart of a monitor setting method provided in an embodiment of the present disclosure.
FIG. 2 shows a schematic diagram of a dock device.
FIG. 3 shows a schematic diagram of an application scenario of a monitor setting method provided in an embodiment of the present disclosure.
FIG. 4 shows a schematic diagram of a location information determination interface in a monitor setting method provided in an embodiment of the present disclosure.
FIG. 5 shows a schematic diagram of an interface for showing related information of a monitor in a monitor setting method provided in an embodiment of the present disclosure.
FIG. 6 shows a block diagram of a monitor setting apparatus provided in an embodiment of the present disclosure.
FIG. 7 shows a block diagram of an electronic device 1900 provided in an embodiment of the present disclosure.

### DETAILED DESCRIPTION

Various exemplary embodiments, features, and aspects of the present disclosure will be described in detail below with reference to the accompanying drawings. The same reference numerals in the drawings represent elements having the same or similar functions. While various aspects of the embodiments are illustrated in the accompanying drawing, the accompanying drawings are not necessarily drawn to scale unless specifically stated.

The word "exemplary" dedicated herein means "used as an example or an embodiment, or is illustrative". Any of the embodiments illustrated herein as "exemplary" is not necessarily interpreted as being superior to or better than other embodiments.

The term "and/or" herein is merely to describe the associations of associated objects, indicating that there may be three kinds of relationships. For example, A and/or B may indicate three situations, i.e., A exists alone, A and B exist simultaneously, or B exists alone. In addition, the term "at least one" herein indicates any one of a plurality or any combination of at least two of a plurality. For example, at least one of A, B, and C may indicate any one or more elements selected from the group consisting of A, B, and C.

In addition, in order to better illustrate the present disclosure, numerous specific details are given in the detailed description below. It should be understood by those skilled in the art that the present disclosure can be implemented without some of the specific details. In some examples, the methods, means, elements, and circuits well known to those skilled in the art are not described in detail in order to highlight the main idea of the present disclosure.

The embodiments of the present disclosure provide a monitor setting method and apparatus, an electronic device, a computer-readable storage medium, and a computer program product. A connection state between a monitor and a dock device is checked. In response to detecting that the monitor is connected to the dock device, device information of the dock device is automatically read from a memory of the dock device. It is determined, on the basis of the device information of the dock device, that the dock device has been replaced, and a location information determination interface is automatically generated. Current location information of the monitor is acquired from the location information determination interface. Target setting information of the monitor matching the current location information is acquired, and the monitor is set according to the target setting information. In this way, the connection state between the monitor and the dock device is automatically checked, and a change in location change is intelligently determined on the basis of the device information of the dock device. The location information determination interface is automatically generated for confirmation performed by a user, so that settings of the monitor are automatically adjusted. The embodiments of the present disclosure significantly reduce manual intervention by the user, reduce the risk of operational error, improve the efficiency and accuracy of changing of monitor settings during patient transport, and optimize clinical work procedures.

In the embodiments of the present disclosure, the dock device does not need a system unit (e.g., a central processing unit, or CPU). Thus, manufacturing costs of the dock device can be significantly reduced, which means that medical institutions can purchase more dock devices to satisfy clinical requirements without bearing an overly heavy economic burden. In addition, the design free of system units further reduces the complexity and maintenance difficulties of the dock device, thereby reducing long-term operation costs.

In addition, the dock device free of any system unit can be easily integrated with different medical devices or systems, thereby improving interconnectivity and interoperability of medical devices. A medical institution can flexibly configure and upgrade a medical device according to actual requirements to satisfy requirements of different departments or patients.

A monitor setting method provided in an embodiment of the present disclosure will be described in detail below with reference to the accompanying drawings.

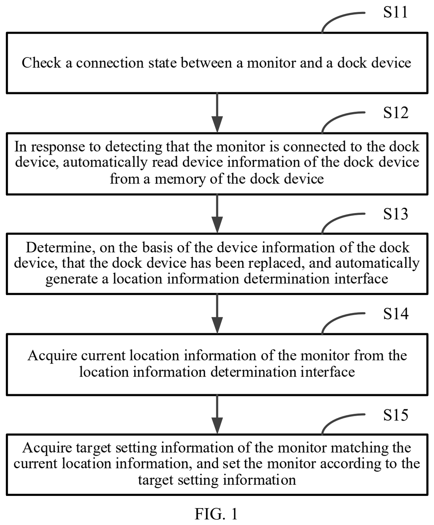

FIG. 1 shows a flowchart of a monitor setting method provided in an embodiment of the present disclosure. In a possible implementation, the monitor setting method may be performed by a monitor setting apparatus. For example, the monitor setting method may be performed by a monitor or another electronic device. In some possible implementations, the monitor setting method may be implemented by a processor by invoking computer-readable instructions stored in a memory. As shown in FIG. 1, the monitor setting method includes step S11 to step S15.

In step S11, a connection state between a monitor and a dock device is checked.

In step S12, in response to detecting that the monitor is connected to the dock device, device information of the dock device is automatically read from a memory of the dock device.

In step S13, it is determined, on the basis of the device information of the dock device, that the dock device has been replaced, and a location information determination interface is automatically generated.

In step S14, current location information of the monitor is acquired from the location information determination interface.

In step S15, target setting information of the monitor matching the current location information is acquired, and the monitor is set according to the target setting information.

The monitor, also referred to as a patient monitor, is a medical device, and is mainly used to monitor vital signs and physiological parameters of a patient. The monitor is typically capable of displaying in real time critical data of a patient such as their heart rate, blood pressure, oxygen saturation, respiratory rate, body temperature, etc., and issuing an alarm when a preset range is exceeded, so that medical personnel can promptly respond. Monitors are widely used in various medical environments such as hospitals, emergency centers, and intensive care units (ICUs), and play a critical role particularly in the processes of patient transport, surgery, rehabilitation, and the like.

A dock device may be used to extend or enhance functions of the monitor. For example, the dock device may include a larger display screen, more connection ports, or other auxiliary devices, thereby facilitating use and extension of a monitor system. In some designs, the dock device may serve as a display and control interface of the monitor, and provide a user interface and data presentation.

In some application scenarios, the dock device may also be referred to as a dock apparatus, a dock station, etc., which is not limited herein.

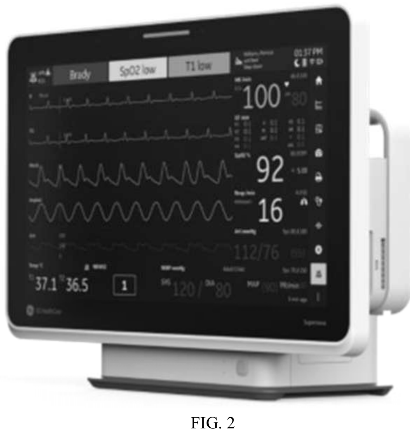

FIG. 2 shows a schematic diagram of a dock device. As shown in FIG. 2, the dock device may include a larger display screen.

In an embodiment of the present disclosure, the connection state between the monitor and the dock device is checked. For example, it may be checked whether physical interfaces between the monitor and the dock device are connected. The monitor may measure parameters such as the level, signal strength, or impedance of the interface via an internal circuit or sensor to ensure the effectiveness of a physical connection.

Once the connection is determined, the monitor may automatically read the device information of the dock device from the memory of the dock device. The device information of the dock device may be information that can be used to uniquely identify the dock device. For example, the device information of the dock device may be an ID, a serial number, or the like of the dock device. In some application scenarios, the device information of the dock device may also be referred to as identification information of the dock device or the like, which is not limited herein. Such configurations, as described above in the embodiments of the present disclosure, can significantly reduce manufacturing costs of the dock device and improve interconnectivity and interoperability of medical devices.

After reading the device information of the currently connected dock device, the monitor may compare the newly read device information with previously recorded device information. If it is found that the device information of the currently connected dock device is inconsistent with the previously recorded device information, it can be determined that the dock device has been replaced. Such replacement may be due to a target patient being transported to a new care unit, and connection to a new dock device is needed.

Upon determining that the dock device has been replaced, the monitor may automatically generate the location information determination interface. The location information determination interface may be used to automatically acquire the current location information of the monitor, or may be used to request the current location information of the monitor from a user. The location information determination interface may be a graphical user interface (GUI), and may include fields that need to be filled out, selected, or confirmed by the user, such as identification information of the care unit, identification information of a bed, etc.

In a possible implementation, the current location information includes: identification information of a current care unit in which the monitor is located.

A care unit is a specific area within a hospital that is acquired by performing division according to patient treatment requirements, and is used to provide a specific type of medical service. Each care unit typically has a specific functional orientation and patient group, such as a general ward, a step-down unit, an intensive care unit (ICU), a pediatric ward, etc.

As one example of this implementation, the identification information of the care unit may include an identifier of the care unit. In this example, each care unit may have a unique identifier for distinguishing and identification within the system. This identifier may be a number, a letter, or a combination of a number and a letter specifically depending on the naming convention of the hospital or medical system.

As another example of this implementation, the identification information of the care unit may include a name or descriptive information of the care unit. The name or descriptive information can more intuitively indicate the location or function thereof. For example, "cardiovascular medical ward", "surgical intensive care unit", and the like.

In another possible implementation, the current location information includes: the identification information of the current care unit in which the monitor is located, and identification information of a current bed of a target patient corresponding to the monitor.

In this implementation, in addition to the identification information of the current care unit in which the monitor is located, the current location information of the monitor further includes the identification information of the current bed of the target patient corresponding to the monitor. The identification information of the bed may be a bed number, a location number, or the like, and can be used to uniquely identify the bed on which the patient is currently lying.

By taking into account the identification information of the current bed of the target patient corresponding to the monitor, the system is allowed to perform finer adjustment according to specific configurations or requirements (e.g., specific monitoring parameters, alarm thresholds, etc.) that may exist for different beds.

It should be noted that, although the current location information of the monitor is described above via the two implementations, those skilled in the art can understand that the present disclosure should not be limited thereto. For example, in other possible implementations, the current location information of the monitor may include at least some of a room number, floor information, a building or area name (e.g., a main hospital area, an east hospital area, etc.), coordinates, identification information for a care team or a primary nurse, etc.

In a possible implementation, the acquiring current location information of the monitor includes: acquiring location information of the dock device; and determining the current location information of the monitor according to the location information of the dock device.

In this implementation, the monitor may automatically acquire the location information of the dock device, and may automatically determine the current location information of the monitor according to the location information of the dock device.

As one example of this implementation, the location information of the dock device may be determined as the current location information of the monitor.

As another example of this implementation, the current location information of the monitor may be determined according to the location information of the dock device and a preset mapping relationship between the location information of the dock device and the location information of the monitor.

In this implementation, the location information of the dock device is automatically identified and acquired, so that the current location information of the monitor can be automatically determined without requiring manual input by a user (for example, medical personnel), thereby improving work efficiency, reducing human error, and enhancing the automation and level of intelligence of medical device management.

As one example of this implementation, the acquiring location information of the dock device includes: reading the location information of the dock device from the memory.

In this example, the dock device may be designed to be capable of storing the location information thereof. Such a design allows the monitor, when connected to the dock device, to automatically read the location information of the dock device from the memory of the dock device to determine the current location information of the monitor.

For example, when each dock device is deployed, location information (e.g., identification information of a care unit, identification information of a bed, a room number, a floor number, etc.) thereof may be recorded in the memory of the dock device. The location information of the dock device may be preset, or may be set by using a certain method (e.g., manual input, automatic identification, etc.) during installation, or may be stored subsequently according to information inputted by the user.

In this example, after the monitor is connected to the dock device, the monitor may attempt to read the location information from the memory of the dock device. After reading the location information of the dock device, the monitor can determine the current location information of the monitor.

In this example, there is no need for manual intervention by a user (e.g., medical personnel), and operation steps are reduced, thereby improving efficiency, and avoiding the problem of inaccurate location information resulting from manual input errors. Once set, the location information of the dock device can be used repeatedly without being frequently changed.

As another example of this implementation, the acquiring location information of the dock device includes: determining the location information of the dock device according to the device information of the dock device and a device information and location mapping table, wherein the device information and location mapping table is acquired from a server end.

The device information and location mapping table may be a database or a data sheet for storing the mapping relationship between the dock device and the corresponding location information thereof. The device information and location mapping table may be located on the server end, and may be accessed by a plurality of clients (e.g., a plurality of monitors).

The monitor may send, to the server end, the read device information of the dock device, or may directly and locally perform matching on the device information and location mapping table downloaded from the server end. The matching process may be to find, according to the device information of the dock device, location information corresponding thereto.

In one example, a server may be arranged in a cloud, so that centralized management of devices can be performed easily, and data loss is not prone to occur. In another example, the server may include a wired or wireless local area network within a medical environment such as a hospital. The local area network of the hospital allows for both security and manageability of devices. For example, if there are a large number of monitors in a hospital, a server in a local area network can analyze and process device information of these monitors together and perform location information matching and/or location allocation, so that the efficiency can be improved, and allocation conflicts are not prone to occur.

After the location information matching the device information of the dock device is found in the device information and location mapping table, the current location information of the monitor may be determined according to the location information of the dock device.

By adopting this example, it is possible to facilitate centralized management and maintenance of the device information and location mapping table, thereby ensuring accuracy and consistency of data. This is particularly important in a medical environment where the patient needs to be moved frequently, as it can significantly reduce the amount of manual setting adjustment work due to changes in locations of devices, improve work efficiency, and reduce human error.

In another possible implementation, the acquiring current location information of the monitor from the location information determination interface includes: acquiring the current location information of the monitor inputted or selected by a user in the location information determination interface.

In this implementation, the location information determination interface may include fields or options for inputting or selecting location information. The user may input or select, in the pop-up location information determination interface, the current location information of the monitor according to a current actual situation. The current location information of the monitor may include identification information (e.g., a name, a number, etc.) of the care unit, a room number, a bed number, etc. The user may provide such information via keyboard input, drop-down menu selection, etc.

In this implementation, the user is allowed to directly input or select the current location information of the monitor in the location information determination interface, so that it is possible to flexibly address location change problems in various complex scenarios.

As one example of this implementation, before the acquiring the current location information of the monitor inputted or selected by a user in the location information determination interface, the method further includes: displaying, in the location information determination interface, identification information of the last care unit in which the monitor was located.

In this example, the monitor may record identification information of the last care unit in which the monitor was located. This means that after each instance of transport is completed, the monitor can record the identification information of the care unit in which the monitor is currently located. When the monitor needs to be transported again, once connection of a new dock device is detected, the identification information of the last care unit can be automatically extracted from records of the monitor and displayed in the location information determination interface.

Displaying of the identification information of the last care unit is of importance to the medical personnel of the current care unit. It provides clear background information to help medical personnel quickly learn the care unit from which a patient was transported. Such a design helps reduce confusion or errors that may be caused by ambiguous information, and improves communication efficiency and accuracy in a transport procedure.

As another example of this implementation, before the acquiring the current location information of the monitor inputted or selected by a user in the location information determination interface, the method further includes: displaying, in the location information determination interface, the identification information of the last care unit in which the monitor was located and identification information of a bed to be confirmed, wherein the bed to be confirmed is automatically allocated by the server end from among free beds.

In this example, in addition to the identification information of the last care unit, identification information of the bed to be confirmed may be further displayed in the location information determination interface. In this example, this bed is not randomly generated, but is automatically allocated by the server end according to currently free beds. The server end may check which beds are free in the current care unit, and automatically allocate a suitable bed to a currently transported patient. This has the advantages of saving the step of manually selecting a bed performed by the user, improving work efficiency, and avoiding the problem of bed conflict.

In this example, the server automatically allocates the bed, thereby further improving the level of intelligence of the system and optimizing a clinical work procedure.

As one example of this implementation, after the acquiring the current location information of the monitor inputted or selected by a user in the location information determination interface, the method further includes: determining the location information of the dock device according to the current location information; storing, in the memory, the location information of the dock device; and/or recording, in the device information and location mapping table, the mapping relationship between the device information and the location information of the dock device, and uploading the updated device information and location mapping table to the server end.

In this example, after the user completes the input or selection, the location information of the dock device currently connected to the monitor may be determined according to the current location information of the monitor provided by the user. For example, the current location information of the monitor may be determined as the location information of the dock device.

In one example, after the location information of the dock device is determined, the location information of the dock device may be stored in the memory of the dock device, so that a monitor subsequently connected to the dock device can read the location information of the dock device from the memory of the dock device.

In one example, after the location information of the dock device is determined, the mapping relationship between the device information and the location information of the dock device may be written into the device information and location mapping table, and the updated device information and location mapping table may be uploaded to the server end, so that the device information and location mapping table accessed by all clients (e.g., a plurality of monitors) are up-to-date.

In an embodiment of the present disclosure, after the current location information of the monitor is acquired from the location information determination interface, the target setting information of the monitor matching the current location information may be acquired, and the monitor may be set according to the target setting information. The target setting information may represent setting information of the monitor matching the current location information of the monitor.

In a possible implementation, the target setting information includes: a target monitoring mode of the monitor.

A monitoring mode refers to a set of predefined parameters and rules adopted by the monitor when performing a monitoring task. Different monitoring modes may be suitable for different patient populations, disease stages, or treatment environments. For example, in an intensive care unit (ICU), the monitor may need to be set to a continuous, highly sensitive monitoring mode to capture any change in vital signs of a patient in real time. In a general ward, the monitoring mode may be a relatively relaxed one.

As one example of this implementation, the monitoring mode of the monitor may include information of the monitoring parameters. The monitor is typically capable of monitoring a variety of physiological parameters including, but not limited to, heart rate, blood pressures (systolic pressure, diastolic pressure, and a mean pressure), respiratory rate, oxygen saturation (SpO2), an electrocardiogram (ECG) waveform, body temperature, etc. During transport of a patient, depending on the requirements of a new care unit, some monitoring parameters may need to be added or deleted, or monitoring precision and frequency of the monitoring parameters may need to be adjusted.

As one example of this implementation, the monitoring mode of the monitor may include an alarm threshold. The alarm threshold may be used to trigger an alarm when a physiological parameter of the patient exceeds a preset range. The alarm threshold may be set for different monitoring parameters, such as the heart rate, the blood pressure, the respiratory rate, etc. When a patient is transferred from one care unit to another care unit, the new care unit may require different alarm threshold settings to accommodate different clinical requirements and patient conditions.

As one example of this implementation, the monitoring mode of the monitor may include display setting information. The display setting information may determine which information to display on a monitor screen and how to display the information. The display setting information may include an oscillogram, a numerical value, a trend diagram, etc. When the location of the patient changes, the new care unit may have different display requirements. For example, a certain monitoring parameter needs to be highlighted, or a screen layout needs to be adjusted to adapt to a different work environment.

In this implementation, when a change in the location of the monitor is detected (e.g., the patient is transported from the general ward to the ICU), the monitoring mode of the monitor may be automatically adjusted, or the user may be prompted to adjust the monitoring mode of the monitor, so as to meet requirements of the new environment. Such automated configuration not only improves work efficiency, but also reduces the risk of human error. The monitor is set to a suitable monitoring mode, so that it can be ensured that physiological parameters of a patient are promptly and accurately monitored, thereby helping a physician to more accurately assess conditions of the patient, adjust a therapeutic regimen, and ultimately improve quality of care and a treatment outcome of the patient.

In other possible implementations, the setting information of the monitor may further include network connection and security information, sensor configuration information, and the like, which are not limited herein.

In a possible implementation, the acquiring target setting information of the monitor matching the current location information includes: reading setting information corresponding to the dock device from the memory, and determining the target setting information of the monitor according to the setting information corresponding to the dock device.

In this implementation, when the monitor is connected to a new dock device, the monitor may attempt to read, from the memory of the dock device, the setting information corresponding thereto. After reading the setting information corresponding to the dock device, the monitor may determine the target setting information thereof according to the setting information corresponding to the dock device, so as to meet requirements of the new location.

In this implementation, the setting information is automatically read from the dock device, and the settings of the monitor are adjusted accordingly, thereby significantly reducing manual intervention by the user, and improving work efficiency. In addition, automatically adjusting the settings can reduce the risk of setting errors resulting from human inattention, thereby improving the accuracy and safety of medical care. This implementation enables the monitor to automatically adjust the settings thereof according to different location information, to accommodate different clinical requirements and environmental changes.

For example, a monitor is originally used in a cardiovascular medical ward and setting information thereof has been optimized for requirements of the ward. When a patient needs to be transferred to a surgical intensive care unit, medical personnel connect the monitor to a dock device of the surgical intensive care unit. In this case, the monitor automatically reads setting information corresponding to the location from the memory of the dock device, and adjusts a monitoring mode thereof accordingly, so as to meet clinical requirements of the surgical intensive care unit.

In another possible implementation, the acquiring target setting information of the monitor matching the current location information includes: determining, according to the current location information and a location and setting information mapping table, the target setting information of the monitor matching the current location information, wherein the location and setting information mapping table is acquired from the server end.

The location and setting information mapping table may be a database or a data sheet, may be stored on the server, and may be used to store a mapping relationship between each location and corresponding setting information of the monitor. Each entry in the location and setting information mapping table may be associated with one location identifier and one set of corresponding monitor setting information.

In this implementation, the monitor may send the acquired current location information to the server, or may directly and locally perform matching on the location and setting information mapping table downloaded from the server. Matching setting information may be looked up in the location and setting information mapping table according to the current location information. If a matching entry is found, the corresponding setting information of the monitor can be extracted.

After the target setting information of the monitor matching the current location information is determined, the target setting information may be applied to the monitor to ensure that the monitor performs proper monitoring according to the new location and environment.

This implementation enables the settings of the monitor to be automatically adjusted according to a change in location, thereby reducing manual intervention by the user, and improving work efficiency. In addition, the location and setting information mapping table is centrally managed, so that the setting information can be easily updated and maintained, thereby ensuring that the monitor always uses the latest configuration suitable for the current location. This implementation also improves the accuracy and reliability of medical monitoring, and reduces the risk of monitoring errors resulting from human error.

In another possible implementation, the acquiring target setting information of the monitor matching the current location information includes: displaying a setting information input interface; and acquiring the target setting information of the monitor inputted or selected by the user in the setting information input interface.

In this implementation, when a change in the location of the monitor is detected and the settings of the monitor need to be adjusted on the basis of this new location information, the setting information input interface may be shown to the user. The setting information input interface may be a graphical user interface (GUI), and may be used to acquire or confirm the target setting information of the monitor.

The user may be prompted to input or select the target setting information of the monitor on the setting information input interface. For example, the user may complete this step by clicking, selecting a drop-down menu, filling out a text box, etc. After the user completes input or selection, the monitor can acquire the target setting information, and the monitor is configured according to the target setting information to meet the requirements of the new location and environment.

In this implementation, the setting information input interface is displayed, and the target setting information of the monitor inputted or selected by the user in the setting information input interface is acquired, thereby allowing the user to flexibly configure the settings of the monitor according to a specific situation.

As one example of this implementation, the displaying a setting information input interface includes: in response to the setting information corresponding to the dock device not being present in the memory and/or setting information matching the current location information not being present in the location and setting information mapping table, displaying the setting information input interface.

In this example, the setting information input interface may be displayed only when the target setting information of the monitor matching the current location information cannot be automatically acquired.

In one example, the setting information input interface may be displayed in response to the setting information corresponding to the dock device not being present in the memory and the setting information matching the current location information not being present in the location and setting information mapping table.

In another example, the setting information input interface may be displayed in response to the setting information corresponding to the dock device not being present in the memory.

In another example, the setting information input interface may be displayed in response to the setting information matching the current location information not being present in the location and setting information mapping table.

If the target setting information cannot be automatically acquired from the memory of the dock device or the location and setting information mapping table, the setting information input interface is displayed, so that the user can manually input or select suitable setting information according to the current location information. This method improves the flexibility and accuracy of setting of the monitor.

In one example, after the acquiring the target setting information of the monitor inputted or selected by the user in the setting information input interface, the method further includes: determining, according to the target setting information, the setting information corresponding to the dock device, and storing, in the memory, the setting information corresponding to the dock device; and/or recording, in the location and setting information mapping table, a mapping relationship between the current location information and the target setting information, and uploading the updated location and setting information mapping table to the server end.

In one example, the setting information corresponding to the dock device may be determined according to the target setting information, and the setting information corresponding to the dock device may be stored in the memory of the dock device. For example, the target setting information may be determined as the setting information corresponding to the dock device. The setting information corresponding to the dock device is stored in the memory of the dock device, so that when the monitor is subsequently connected to the dock device, the setting information corresponding to the dock device may be automatically read from the memory of the dock device. Therefore, the target setting information of the monitor is automatically determined, and manual setting does not need to be performed again, thereby improving the intelligence of the system and the user experience.

In another example, the mapping relationship between the current location information and the target setting information may be recorded in the location and setting information mapping table, and the updated location and setting information mapping table may be uploaded to the server end. In this way, a plurality of clients (e.g., a plurality of monitors) may share the location and setting information mapping table, so that the setting information is centrally managed and synchronously updated.

In a possible implementation, the method further includes: determining, on the basis of the device information of the dock device, that the dock device has not been replaced, and skipping generating the location information determination interface.

In this implementation, the monitor may check the device information of the currently connected dock device at a preset frequency or according to a specific event (e.g., reconnection of the dock device), and compare the device information with previously recorded information. If the device information of the dock device has not changed, it can be considered that the dock device has not been replaced, that is, the dock device is still the previously connected dock device. If it is determined that the dock device has not been replaced, the location information determination interface may not be generated.

This implementation can improve the efficiency of setting of the monitor and the user experience. Whether the dock device has been replaced is intelligently identified, so that unnecessary user interaction can be avoided, thereby simplifying the operation flow and reducing the operation load of the user.

In a possible implementation, the method further includes: in response to a state of the target patient corresponding to the monitor being changed to discharged, setting the monitor according to default setting information.

In this implementation, when the state of the target patient corresponding to the monitor (i.e., the patient currently monitored by the monitor) is changed from a hospitalized state to a discharged state, the system can detect such a change in state. Such detection may be based on an internal information system (e.g., a HIS system) of the hospital, or may be performed via a certain logical determination performed by the monitor (e.g., detecting that patient information is removed or modified).

In this implementation, default setting information may be preset. The default setting information may refer to standard configurations or settings employed by the monitor when there is no specific patient information or special requirements. The default setting information may include screen brightness, a volume, an alarm threshold, default ranges of displayed parameters (e.g., the heart rate, the blood pressure, etc.), etc.

In this implementation, in response to detecting that the patient is discharged, an automated procedure may be triggered to reconfigure the monitor according to the preset default setting information. This process may be performed automatically via software instructions without human intervention.

In this implementation, the settings of the monitor are automatically adjusted according to the change in the state of the patient, thereby reducing manual operation steps of medical personnel and improving work efficiency. By means of the preset default setting information, medical risks caused by human inattention or wrong configuration are reduced.

For example, after a patient has been monitored by a monitor for several days, a physician decides to discharge the patient. In this case, the information system of the hospital automatically updates the state of the patient, and triggers an automatic setting adjustment mechanism of the monitor. The monitor automatically returns to a default setting state so as to be prepared for the next patient. In this way, when a new patient is connected to the monitor, medical personnel can directly start the work of monitoring without needing to manually adjust the parameters of the monitor.

In another possible implementation, the method further includes: in response to the state of the target patient corresponding to the monitor being changed to discharged, skipping resetting the monitor, for example, causing the monitor to remain in a current monitoring mode.

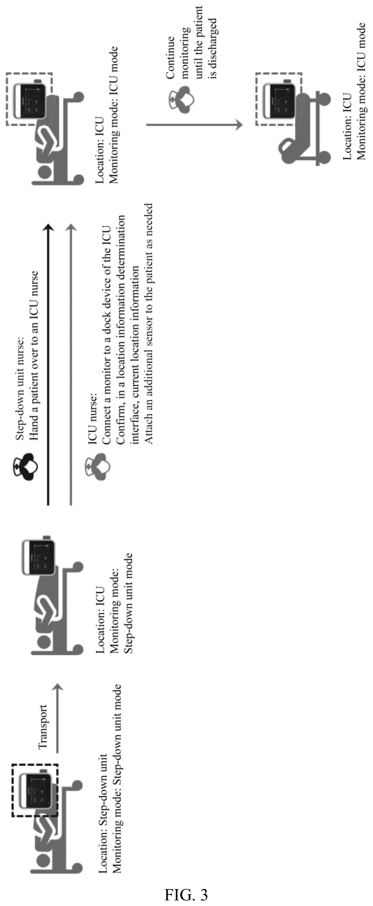

The monitor setting method provided in the embodiments of the present disclosure will be described below via a specific application scenario. FIG. 3 shows a schematic diagram of an application scenario of a monitor setting method provided in an embodiment of the present disclosure.

As shown in FIG. 3, a step-down unit nurse is about to transport a patient to an ICU. In this case, a monitor of the patient is connected to a dock device of a step-down unit, and is in a step-down unit mode. The step-down unit nurse hands the patient over to an ICU nurse, and informs the ICU nurse of basic information and transport requirements of the patient.

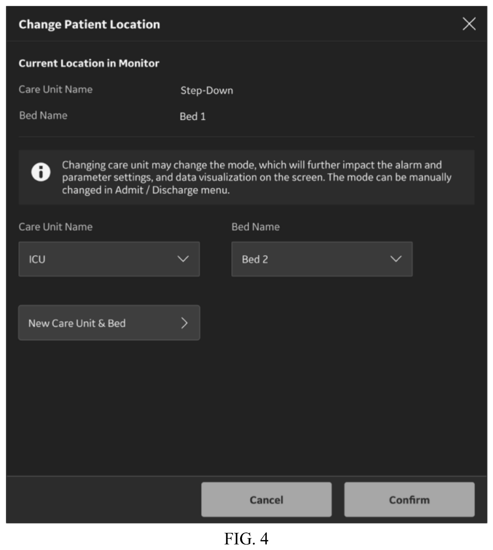

The ICU nurse pushes the patient into the ICU, and prepares to connect the monitor to a dock device of the ICU. When the monitor is inserted into the dock device of the ICU, the monitor automatically reads device information of the dock device from a memory of the dock device. The monitor may determine, on the basis of the device information of the dock device, that the dock device has been replaced, and automatically pop up a location information determination interface. The monitor may read location information "ICU" of the dock device from the memory of the dock device, and may determine the location information "ICU" of the dock device as current location information of the monitor. The ICU nurse may confirm, in the location information determination interface, the current location information of the monitor. FIG. 4 shows a schematic diagram of a location information determination interface in a monitor setting method provided in an embodiment of the present disclosure.

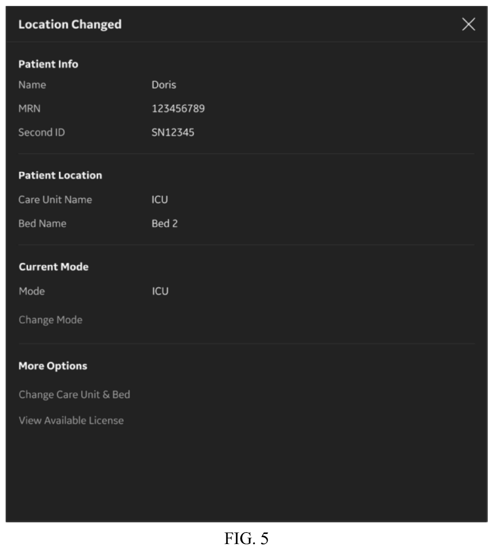

The monitor may acquire a location and setting information mapping table from a server end, and determine, according to the current location information "ICU" of the monitor and the location and setting information mapping table, a target monitoring mode "ICU mode" of the monitor matching the current location information "ICU". That is, the monitor may automatically switch the monitoring mode from the step-down unit mode to the ICU mode. FIG. 5 shows a schematic diagram of an interface for showing related information of a monitor in a monitor setting method provided in an embodiment of the present disclosure. In an example shown in FIG. 5, information about the patient, the current location information of the monitor, and the monitoring mode of the monitor may be shown.

Depending on specific conditions of the patient, the ICU nurse may also attach, to the patient, additional sensors for, e.g., blood pressure monitoring, oxygen saturation monitoring, etc.

The monitor in the ICU mode continuously monitors vital signs of the patient. When the patient is discharged from the ICU or transferred to another department, the ICU nurse pulls the monitor out of the dock device of the ICU. If the system is configured to "return to the previously used mode", the monitor automatically reverts to the ICU mode or another mode specified by the user. If the system is configured to be in a "default mode", the monitor reverts to default settings.

It can be understood that all the foregoing method embodiments mentioned in the present disclosure may be combined with each other to form a combined embodiment without departing from the principle or logic, which will not be described in detail in the present disclosure due to limited space. It can be understood by those skilled in the art that, in the above methods described in the detailed embodiments, the specific order in which each step is performed should be determined by the function and possible inherent logic thereof.

In addition, the present disclosure also provides a monitor setting apparatus, an electronic device, a computer-readable storage medium, and a computer program product, all of which can be used to implement any one of the monitor setting methods provided by the present disclosure. For corresponding technical solutions and technical effects, reference may be made to corresponding descriptions in the method sections, and details will not be described herein again.

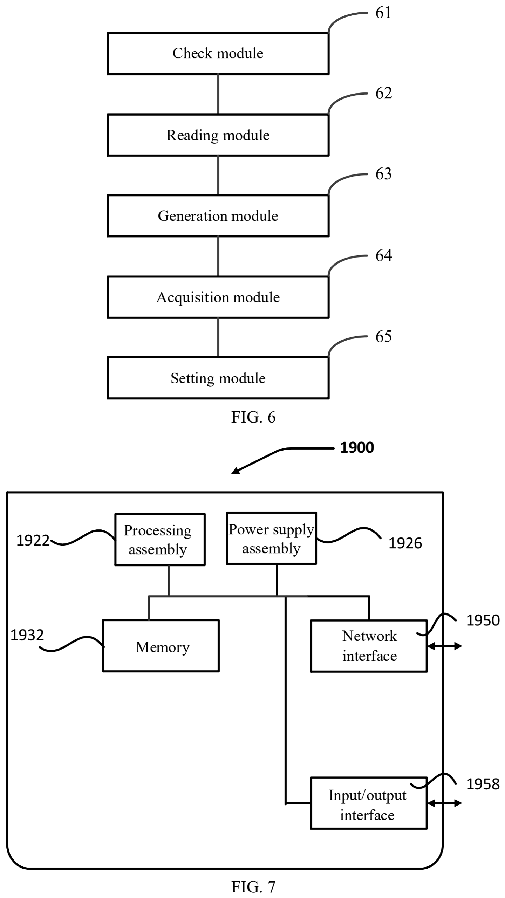

FIG. 6 shows a block diagram of a monitor setting apparatus provided in an embodiment of the present disclosure. As shown in FIG. 6, the monitor setting apparatus includes:
a check module 61, configured to check a connection state between a monitor and a dock device;
a reading module 62, configured to, in response to detecting that the monitor is connected to the dock device, automatically read device information of the dock device from a memory of the dock device;
a generation module 63, configured to determine, on the basis of the device information of the dock device, that the dock device has been replaced, and automatically generate a location information determination interface;
an acquisition module 64, configured to acquire current location information of the monitor from the location information determination interface; and
a setting module 65, configured to acquire target setting information of the monitor matching the current location information, and set the monitor according to the target setting information.

In a possible implementation, the current location information includes:
identification information of a current care unit in which the monitor is located;
   or,
the identification information of the current care unit in which the monitor is located, and identification information of a current bed of a target patient corresponding to the monitor.

In a possible implementation, the target setting information includes:
a target monitoring mode of the monitor.

In a possible implementation, the acquisition module 64 is configured to:
acquire location information of the dock device; and
determine the current location information of the monitor according to the location information of the dock device.

In a possible implementation, the acquisition module 64 is configured to:
read the location information of the dock device from the memory;
   or,
determine the location information of the dock device according to the device information of the dock device and a device information and location mapping table, wherein the device information and location mapping table is acquired from a server end.

In a possible implementation, the acquisition module 64 is configured to:
acquire the current location information of the monitor inputted or selected by a user in the location information determination interface.

In a possible implementation, the apparatus further includes a display module, and the display module is configured to:
display, in the location information determination interface, identification information of the last care unit in which the monitor was located;
   or,
display, in the location information determination interface, the identification information of the last care unit in which the monitor was located and identification information of a bed to be confirmed, wherein the bed to be confirmed is automatically allocated by the server end from among free beds.

In a possible implementation, the apparatus further includes:
a determination module, configured to determine the location information of the dock device according to the current location information; and
a first storage module, configured to store, in the memory, the location information of the dock device; and/or record, in the device information and location mapping table, a mapping relationship between the device information and the location information of the dock device, and upload the updated device information and location mapping table to the server end.

In a possible implementation, the setting module 65 is configured to:
read setting information corresponding to the dock device from the memory, and determine the target setting information of the monitor according to the setting information corresponding to the dock device;
   or,
determine, according to the current location information and a location and setting information mapping table, the target setting information of the monitor matching the current location information, wherein the location and setting information mapping table is acquired from the server end.

In a possible implementation, the setting module 65 is configured to:
display a setting information input interface; and
acquire the target setting information of the monitor inputted or selected by the user in the setting information input interface.

In a possible implementation, the setting module 65 is configured to:
in response to the setting information corresponding to the dock device not being present in the memory and/or setting information matching the current location information not being present in the location and setting information mapping table, display the setting information input interface.

In a possible implementation, the apparatus further includes a second storage module, and the second storage module is configured to:
determine, according to the target setting information, the setting information corresponding to the dock device, and store, in the memory, the setting information corresponding to the dock device;
   and/or,
record, in the location and setting information mapping table, a mapping relationship between the current location information and the target setting information, and upload the updated location and setting information mapping table to the server end.

In a possible implementation, the setting module 65 is further configured to:
in response to a state of the target patient corresponding to the monitor being changed to discharged, set the monitor according to default setting information.

In a possible implementation, the generation module 63 is further configured to:
determine, on the basis of the device information of the dock device, that the dock device has not been replaced, and skip generating the location information determination interface.

In some embodiments, the function of the apparatus provided in the embodiments of the present disclosure or the modules included in the apparatus may be configured to perform the method described in the foregoing method embodiments, and for specific implementation and technical effects thereof, reference may be made to the description of the foregoing method embodiments, which are not described herein again for brevity.

An embodiment of the present disclosure further provides a computer-readable storage medium, having computer program instructions stored thereon, wherein the computer program instructions, when executed by a processor, implement the above method. The computer-readable storage medium may be a non-volatile computer-readable storage medium or a volatile computer-readable storage medium.

An embodiment of the present disclosure further provides a computer program, including computer-readable code, wherein when the computer-readable code is run in an electronic device, a processor in the electronic device performs the above method.

An embodiment of the present disclosure further provides a computer program product, including computer-readable code or a non-volatile computer-readable storage medium bearing computer-readable code, wherein when the computer-readable code is run in an electronic device, a processor in the electronic device performs the above method.

An embodiment of the present disclosure further provides an electronic device, including: one or more processors; and a memory for storing executable instructions, wherein the one or more processors are configured to invoke the executable instructions stored by the memory to perform the above method.

The electronic device may be provided as a monitor or a device in another form.

FIG. 7 shows a block diagram of an electronic device 1900 provided in an embodiment of the present disclosure. For example, the electronic device 1900 may be provided as a monitor. Referring to FIG. 7, the electronic device 1900 includes a processing assembly 1922 that further includes one or more processors, and a memory resource represented by a memory 1932 for storing instructions that can be executed by the processing assembly 1922, such as an application. The application stored in the memory 1932 can include one or more modules each corresponding to a set of instructions. In addition, the processing assembly 1922 is configured to execute instructions to perform the above method.

The electronic device 1900 can further include a power supply assembly 1926 configured to perform power management of the electronic device 1900, a wired or wireless network interface 1950 configured to connect the electronic device 1900 to a network, and an input/output (I/O) interface 1958. The electronic device 1900 can operate on the basis of an operating system stored in the memory 1932, for example, a Microsoft server operating system (Windows Server^{™}), a graphical user interface-based operating system (Mac OS X^{™}) provided by Apple Inc., a multi-user multi-process computer operating system (Unix^{™}), a free and open source Unix-like operating system (Linux^{™}), an open source Unix-like operating system (FreeBSD^{™}) or the like.

In an exemplary embodiment, a non-volatile computer-readable storage medium is also provided, such as the memory 1932 including computer program instructions that are executable by the processing assembly 1922 of the electronic device 1900 to perform the above method.

The present disclosure may be a system, a method and/or a computer program product. The computer program product may include a computer-readable storage medium, having computer-readable program instructions thereon for causing a processor to implement various aspects of the present disclosure.

The computer-readable storage medium can be a tangible device that can hold and store instructions used by an instruction execution device. The computer-readable storage medium may be, for example, but is not limited to, an electrical storage device, a magnetic storage device, an optical storage device, an electromagnetic storage device, a semiconductor memory device, or any suitable combination thereof. More specific examples (a non-exhaustive list) of the computer-readable storage medium include: a portable computer disk, a hard disk, a random access memory (RAM), a read only memory (ROM), a erasable programmable read only memory (EPROM or flash memory), a static random access memory (SRAM), a portable compact disc read-only memory (CD ROM), a digital versatile disk (DVD), a memory stick, a floppy disk, mechanical coding equipment, such as a punch card with instructions stored thereon or a structure of bumps within recessions, and any suitable combination thereof. The computer-readable storage medium used herein is not interpreted as transient signals themselves, such as radio waves or other freely propagated electromagnetic waves, electromagnetic waves propagated through a waveguide or other transmission media (e.g., light pulses passing through a fiber optic cable), or electrical signals transmitted through electric wires.

The computer-readable program instructions described herein may be downloaded from a computer-readable storage medium to various computing/processing devices or downloaded to an external computer or external storage device via a network such as the Internet, a local area network, a wide area network and/or a wireless network. The network may include copper transmission cables, fiber transmission, wireless transmission, routers, firewalls, switches, gateway computers, and/or edge servers. A network adapter card or a network interface in each computing/processing device receives computer-readable program instructions from the network and forwards the computer-readable program instructions, for storing them in a computer-readable storage medium in each computing/processing device.

Computer program instructions for performing the operations of the present disclosure can be assembly instructions, instruction set architecture (ISA) instructions, machine instructions, machine related instructions, microcode, firmware instructions, state setting data, or source code or object code written in any combination of one or more programming languages, the programming language including object oriented programming languages such as Smalltalk, C++, and the like, and conventional procedural programming languages such as the "C" language or similar programming languages. The computer-readable program instructions can be executed entirely or partly on a user computer, executed as a stand-alone software package, executed partly on a user computer and partly on a remote computer, or executed entirely on a remote computer or server. In the case of a remote computer, the remote computer may be connected to the user's computer through any kind of network, including a local area network (LAN) or a wide area network (WAN). Alternatively, it can be connected to an external computer (for example, using an Internet service provider to connect via the Internet). In some embodiments, an electronic circuit, for example, a programmable logic circuit, a field-programmable gate array (FPGA), or a programmable logic array (PLA), may execute the computer-readable program instructions by utilizing state information of the computer-readable program instructions to personalize the electronic circuit, in order to implement various aspects of the present disclosure.

The aspects of the present disclosure are described herein with reference to the flowcharts and/or block diagrams of the methods, apparatuses (systems), and computer program products according to the embodiments of the present disclosure. It should be understood that each block of the flowcharts and/or block diagrams and combinations of various blocks in the flowcharts and/or block diagrams can be implemented by computer-readable program instructions.

These computer-readable program instructions may be provided to a processor of a general-purpose computer, a special-purpose computer, or other programmable data processing apparatuses, to produce a machine, so that these instructions, when executed by the processor of the computer or other programmable data processing apparatuses, produce an apparatus for implementing the functions/actions specified in one or more blocks of the flowcharts and/or block diagrams. Also, these computer-readable program instructions may be stored in a computer-readable storage medium. These instructions cause a computer, a programmable data processing device, and/or other devices to work in a specific manner. Thus, the computer-readable medium storing the instructions includes an artifact, including instructions that implement various aspects of the functions/actions specified in one or more the flowcharts and/or block diagrams.

The computer-readable program instructions may also be loaded onto a computer, other programmable data processing apparatuses, or other devices, such that the computer, other programmable data processing apparatuses, or other devices perform a series of operational steps, to generate a computer-implemented process, such that the functions/actions specified in one or more of the flowcharts and/or block diagrams are implemented by the instructions executed on the computer, other programmable data processing apparatuses, or other devices.

The flowcharts and block diagrams in the accompanying drawings illustrate system architectures, functions, and operations of possible implementations of the system, method, and computer program product according to a plurality of embodiments of the present disclosure. In this regard, each block in the flowcharts or block diagrams may represent a portion of a module, program segment, or instruction that contains one or more executable instructions for implementing the specified logical functions. In some alternative implementations, the functions denoted in the blocks can also occur in a different order than that illustrated in the drawings. For example, two consecutive blocks can actually be performed substantially in parallel, and sometimes can also be performed in a reverse order, depending upon the functions involved. It should also be noted that each block of the block diagrams and/or flowcharts, and combinations of blocks in the block diagrams and/or flowcharts can be implemented in a dedicated hardware-based system that performs the specified function or action, or can be implemented by a combination of dedicated hardware and computer instructions.

The computer program product may be embodied in hardware, software, or a combination thereof. In an alternative embodiment, the computer program product is embodied as a computer storage medium, and in another alternative embodiment, the computer program product is embodied as a software product, such as a software development kit (SDK) or the like.

The above description of the various embodiments tends to emphasize differences between the various embodiments, and reference may be made therebetween for the same features or similarities thereof, which will not be described in detail herein for the sake of brevity.

If the technical solution of the embodiments of the present disclosure involves personal information, a product to which the technical solution of the embodiments of the present disclosure is applied has explicitly performed notification of a personal information processing rule before processing the personal information, and has acquired individual self-consent. If the technical solution of the embodiments of the present disclosure involves sensitive personal information, a product to which the technical solution of the embodiments of the present disclosure is applied has acquired individual consent before processing the sensitive personal information, and also satisfies the requirement of "explicit consent". For example, at a personal information collection apparatus such as a camera, a clear and obvious sign is provided to state that a personal information collection range has been entered, and personal information will be collected. If a person voluntarily enters the collection range, it is regarded as consent to collect personal information thereof. Alternatively, if notification of a personal information processing rule is performed on a personal information processing apparatus by using an obvious sign/information, personal authorization is acquired by means of pop-up information or by inviting the individual to upload personal information thereof. The personal information processing rule may include information such as a personal information handler, a personal information processing purpose, a processing method, and a type of personal information to be processed.

The embodiments of the present disclosure have been described above. The foregoing description is illustrative rather than limiting, and is not limited to the disclosed embodiments. Many modifications and variations are apparent to those of ordinary skill in the art without departing from the scope and spirit of the embodiments illustrated. The selection of terms used herein is intended to best explain the principles, practical applications, or improvements to the techniques in the market of the embodiments, or to enable others of ordinary skill in the art to understand the embodiments disclosed herein.

## Claims

1. A monitor setting method, **characterized by** comprising:
checking a connection state between a monitor and a dock device;
in response to detecting that the monitor is connected to the dock device, automatically reading device information of the dock device from a memory of the dock device;
determining, on the basis of the device information of the dock device, that the dock device has been replaced, and automatically generating a location information determination interface;
acquiring current location information of the monitor from the location information determination interface; and
acquiring target setting information of the monitor matching the current location information, and setting the monitor according to the target setting information.

2. The method according to claim 1, wherein the current location information comprises:
identification information of a current care unit in which the monitor is located;
or,
the identification information of the current care unit in which the monitor is located, and identification information of a current bed of a target patient corresponding to the monitor.

3. The method according to claim 1, wherein the target setting information comprises:
a target monitoring mode of the monitor.

4. The method according to any one of claims 1 to 3, wherein the acquiring current location information of the monitor comprises:
acquiring location information of the dock device; and
determining the current location information of the monitor according to the location information of the dock device.

5. The method according to claim 4, wherein the acquiring location information of the dock device comprises:
reading the location information of the dock device from the memory;
or,
determining the location information of the dock device according to the device information of the dock device and a device information and location mapping table, wherein the device information and location mapping table is acquired from a server end.

6. The method according to any one of claims 1 to 3, wherein the acquiring current location information of the monitor from the location information determination interface comprises:
acquiring the current location information of the monitor inputted or selected by a user in the location information determination interface.

7. The method according to claim 6, wherein before the acquiring the current location information of the monitor inputted or selected by a user in the location information determination interface, the method further comprises:
displaying, in the location information determination interface, identification information of the last care unit in which the monitor was located;
or,
displaying, in the location information determination interface, the identification information of the last care unit in which the monitor was located and identification information of a bed to be confirmed, wherein the bed to be confirmed is automatically allocated by a server end from among free beds.

8. The method according to claim 6, wherein after the acquiring the current location information of the monitor inputted or selected by a user in the location information determination interface, the method further comprises:
determining location information of the dock device according to the current location information;
storing, in the memory, the location information of the dock device; and/or recording, in a device information and location mapping table, a mapping relationship between the device information and the location information of the dock device, and uploading the updated device information and location mapping table to a server end.

9. The method according to any one of claims 1 to 3, wherein the acquiring target setting information of the monitor matching the current location information comprises:
reading setting information corresponding to the dock device from the memory, and determining the target setting information of the monitor according to the setting information corresponding to the dock device;
or,
determining, according to the current location information and a location and setting information mapping table, the target setting information of the monitor matching the current location information, wherein the location and setting information mapping table is acquired from a server end.

10. The method according to any one of claims 1 to 3, wherein the acquiring target setting information of the monitor matching the current location information comprises:
displaying a setting information input interface; and
acquiring the target setting information of the monitor inputted or selected by a user in the setting information input interface.

11. The method according to claim 10, wherein the displaying a setting information input interface comprises:
in response to setting information corresponding to the dock device not being present in the memory and/or setting information matching the current location information not being present in a location and setting information mapping table, displaying the setting information input interface.

12. The method according to claim 11, wherein after the acquiring the target setting information of the monitor inputted or selected by a user in the setting information input interface, the method further comprises:
determining, according to the target setting information, setting information corresponding to the dock device, and storing, in the memory, the setting information corresponding to the dock device;
and/or,
recording, in the location and setting information mapping table, a mapping relationship between the current location information and the target setting information, and uploading the updated location and setting information mapping table to a server end.

13. The method according to any one of claims 1 to 3, further comprising:
in response to a state of a target patient corresponding to the monitor being changed to discharged, setting the monitor according to default setting information.

14. The method according to any one of claims 1 to 3, further comprising:
determining, on the basis of the device information of the dock device, that the dock device has not been replaced, and skipping generating the location information determination interface.
